# EUROPEAN PATENT APPLICATION

(11) **EP 2 550 943 A1**
(43) Date of publication of application: **30.01.2013**
(21) Application number: 11759305.3
(22) Date of filing: 17.03.2011
(51) Int. Cl.: A61F 2/82, A61F 2/06, A61L 31/00

(54) **REPAIR MATERIAL FOR CONDUIT OF LIVING BODY**

(30) Priority: 23.03.2010 JP 2010066283
(71) Applicant: Terumo Kabushiki Kaisha, Tokyo 151-0072 (JP)
(72) Inventor: TANI Kazuyoshi, Fujinomiya-shi Shizuoka 418-0015 (JP); HASHIMOTO Hiromitsu, Ashigarakami-gun Kanagawa 259-0151 (JP); SOMA Katsuaki, Fujinomiya-shi Shizuoka 418-0015 (JP); ITO Takenari, Fujinomiya-shi Shizuoka 418-0015 (JP)
(74) Representative: Dossmann, Gérard
(86) International application number: PCT/JP2011/056392
(87) International publication number: WO 2011/118499

(57) **Abstract**

Disclosed herein is a stent graft (10, 10a, 10b, 20, 30, 30a, 40) as a repairing material for lumens such as aorta and other blood vessels and trachea) of a living body. It includes warp yarns (14) of plastic material and weft yarns (12, 42) including filaments of at least one of shape-memory plastic, shape-memory alloy, and super-elastic metal, the warp yarns and weft yarns being interwoven into a tubular shape such that the weft yarns (12, 42) extend in the circumferential direction and the warp yarns (14) extend in the axial direction, with either or both of the warp yarns (14) and weft yarns (12, 42) being formed from yarns capable of swelling by body liquid or from yarns with coating (16) capable of swelling by body fluid.

## Description

### Technical Field

The present invention relates to a repairing material for lumens of a living body, the repairing material being in a tubular shape formed by interweaving warp yarns and weft yarns and being intended to repair lumens, such as blood vessels, of a living body.

### Background Art

Surgical operations involving partial replacement of blood vessels with artificial ones are often practiced for treatment of such diseases as aneurysm in the chest or abdomen. Unfortunately, they tend to be highly invasive to the patient. This disadvantage is being overcome by placing a stent graft as a blood vessel repairing material in the impaired blood vessel remaining unremoved.

The stent graft is usually composed of a cylindrical tube (graft) of fabric woven from plastic yarns such as polyester and a skeleton (stent) in a ring or zigzag shape formed from wires of stainless steel or nickel-titanium alloy, with the latter being stitched and fixed to the inside or outside of the former (see JP-T-2007-518532, for example). Popular among current stent grafts are self-expandable ones which do not need balloons.

### Summary of the Invention

The above-mentioned stent graft presents difficulties in being made sufficiently thin by folding up in such a direction as to reduce its outside diameter at the time of insertion into the blood vessel, because it has a diameter increased by the skeleton and the stitching yarns. The stent graft with such an increased diameter needs a delivery sheath with a large diameter for its delivery to the desired position. This wastes the effort to reduce invasion to the patient. In addition, the skeleton constituting the stent graft tends to cause local force concentration at the part where it comes into contact with the fabric, thereby adversely affects the wall of the blood vessel that is in contact with the stent graft, or breaking the fabric.

The present invention was completed to address the foregoing problems involved in conventional technologies. It is an object of the present invention to provide a repairing material for lumens of a living body, which can be made sufficiently thin and free of local force concentration, and hence which is less invasive to the patient than the conventional one.

According to the present invention, the repairing material for lumens of a living body includes warp yarns of plastic material and weft yarns including filaments of at least one of shape-memory plastic, shape-memory alloy, and super-elastic metal, the warp yarns and weft yarns being interwoven into a tubular shape such that the weft yarns extend in the circumferential direction and the warp yarns extend in the axial direction, with either or both of the warp yarns and weft yarns being formed from yarns capable of swelling by body liquid or having coating capable of swelling by body fluid.

The repairing material for lumens of a living body, which is constructed as mentioned above, is characterized in that the weft yarns, which extend in the circumferential direction of the tube, include filaments formed from at least one of shape-memory plastic, shape-memory alloy, and super-elastic metal, so that the weft yarns have sufficient resilience. Consequently, the weft yarns function as the conventional skeleton (stent) of metal wire attached to the inside or outside of the tube (graft), and this eliminates the necessity of the skeleton. The repairing material free of skeleton has a thin wall and a small diameter corresponding to the thickness of the skeleton and the thickness of the yarn used to stitch the skeleton to the fabric. Because of this feature, it can be easily folded up small at the time of insertion into the body. This helps reduce invasion to the patient. The folded repairing material is smoothly expanded by the elastic force of the weft yarns. The weft yarns serving as the skeleton apply an approximately even force to the repairing material. This ensures the safety of the vessel wall in contact with the stent graft and also prevents the fabric from breaking.

Moreover, either or both of the warp yarns and the weft yarns are formed from filaments capable of swelling by the body fluid or formed from filaments with coating capable of swelling by the body fluid. This feature permits the repairing material to be constructed of a less number of warp yarns and weft yarns than usual. The resulting repairing material can be made thin when folded up. In other words, even though the number of warp yarns and weft yarns is reduced, the warp yarns and weft yarns swell in the living body, thereby completely closing interstices between them and allowing the repairing material to function satisfactorily.

The weft yarns may have an irregular wavy shape along the axial direction composed of convex and concave curves along the circumference. This structure permits the repairing material to be folded up more easily. This structure also permits the repairing material to be folded up from its expanded form into its compressed form with a smaller deformation ratio than the ordinary structure of circularly curved weft yarns. As the result, the weft yarns decrease in rebound resilience when the repairing material is held in the sheath of a catheter, which leads to an easy release of the repairing material from the sheath.

The weft yarns are composed of two kinds of yarns, with the first one being formed from at least one of shape-memory plastic, shape-memory alloy, and super-elastic metal, and the second one being formed from a plastic material. They may be alternately arranged in a specific ratio to be interwoven with the warp yarns. The alternate arrangement of the yarns of the first and second kinds prevents the metallic yarns from lying side by side and hence slipping from each other. In addition, the mixing of the yarns of the first kind which are elastic with the yarns of the second kind in a specific ratio permits the repairing material to be properly controlled in expandability and compressibility.

The mixing ratio of the yarns of the first kind and the yarns of the second kind may be such that they are arranged alternately in a ratio of one to one or one or more to two or more.

It is desirable that the ratio of the yarns of the first kind to the yarns of the second kind be maintained over the entire length of the repairing material. The arrangement of yarns in this manner prevents the metallic yarns from lying side by side and hence from slipping from each other easily. It also ensures the uniform expandability and compressibility over the entire length of the repairing material.

The weft yarns may be arranged in such a way that the ratio of the yarns of the first kind to the yarns of the second kind is higher in near the ends than at the intermediate position along the axis of the repairing material. The arrangement of weft yarns in this manner makes the vicinity of the ends more elastic than the intermediate part of the repairing material. With the enhanced elasticity at its ends, the repairing material smoothly expands at the desired position in the lumen to ensure its placement. With the reduced expanding force at the intermediate position, the repairing material can be easily folded up owing to the decreased resistance to compression encountered when the repairing material is folded up.

It is also desirable that at least either of the ends of the repairing material is formed from the yarns of the first kind, so that the end exhibits a sufficient expanding force. This further ensures the placement of the repairing material in the lumen.

It is desirable to construct the repairing material in such a way that the ends thereof are formed from more resilient weft yarns and the intermediate part thereof is formed from less resilient weft yarns. The repairing material constructed in this manner exhibits a greater resilience near the ends thereof.

To achieve this object, it is desirable to use thicker or more resilient weft yarns near the ends than at the intermediate part of the repairing material.

The repairing material should preferably have more than one hook on the circumference of at least one end thereof. Such hooks bite into the wall of the blood vessel, thereby ensuring the placement of the repairing material in the lumen.

The repairing material according to the present invention is a woven fabric in tubular form in which the weft yarns in the circumferential direction are at least one kind of yarns of shape-memory plastic, shape-memory alloy, and super-elastic metal, having sufficient elasticity. Such weft yarns function as the skeleton, which are made of metallic wires and attached to the inside or outside of the tube (graft) in the conventional repairing material. Therefore, this structure eliminates the necessity of the skeleton, thereby reducing the thickness and diameter of then tube corresponding to the thickness of the skeleton and the yarns used to stitch the skeleton to the fabric.

The effect of the foregoing structure is that the repairing material can be easily folded up into a thin form at the time of insertion into the living body (with reduced invasion to the patient) and can also be fully expanded in the lumen owing to the elastic force of the weft yarns. The weft yarns serving as the skeleton exert an approximately uniform force to the repairing material. This leads to the application of a local strong force, which ensures the safety of the vessel wall in contact with the stent graft and prevents the fabric from breaking.

As mentioned above, either or both of the warp yarns and weft yarns are formed from yarns capable of swelling by body fluid or from yarns with coating capable of swelling by body fluid. This structure permits the repairing material to be formed from a less number of warp yarns and weft yarns than usual, which means that the repairing material can be folded up into a thin body. The warp yarns and weft yarns in a reduced number can completely close their interstices, thereby allowing the repairing material placed in the lumen to work satisfactorily.

### Brief Description of the Drawings

[Fig. 1]

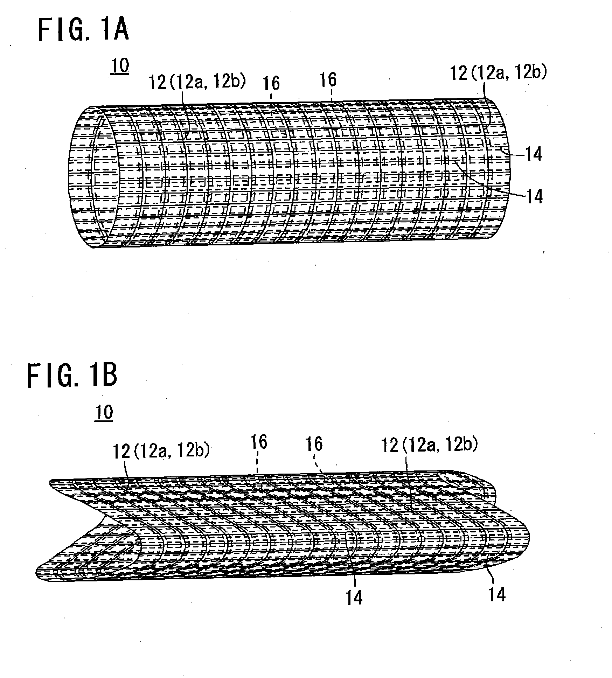

   Fig. 1A is a perspective view showing a stent graft in its expanded state, which is the repairing material for lumens of a living body pertaining to the first embodiment of the present invention. Fig. 1B is a perspective view showing the stent graft shown in Fig. 1A in its folded state.
[Fig. 2]

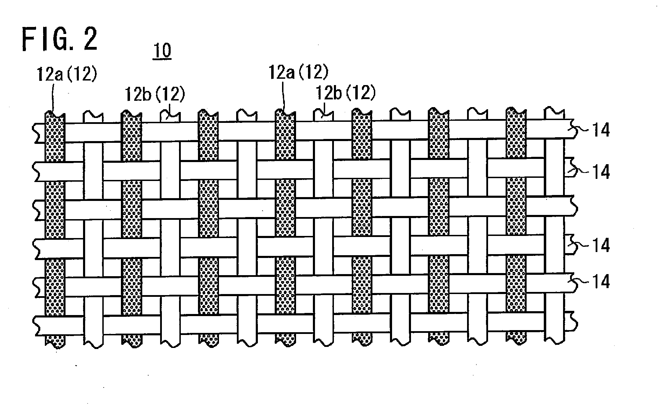

   Fig. 2 is a partly enlarged side view of the stent graft shown in Fig. 1A.
[Fig. 3]

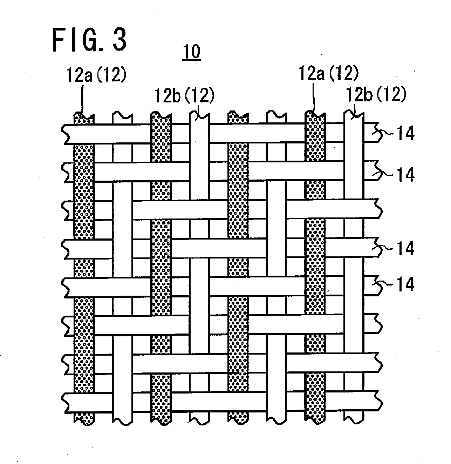

   Fig. 3 is a diagram illustrating one method of interweaving for the stent graft.
[Fig. 4]

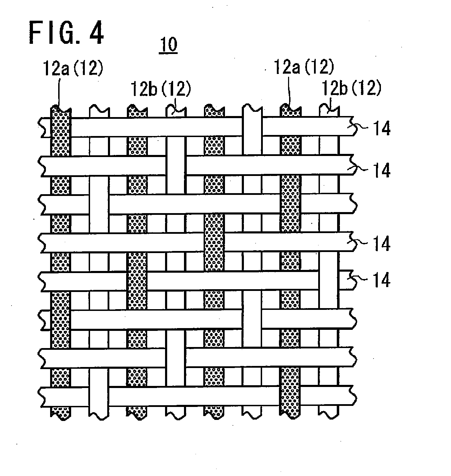

   Fig. 4 is a diagram illustrating another method of interweaving for the stent graft.
[Fig. 5]

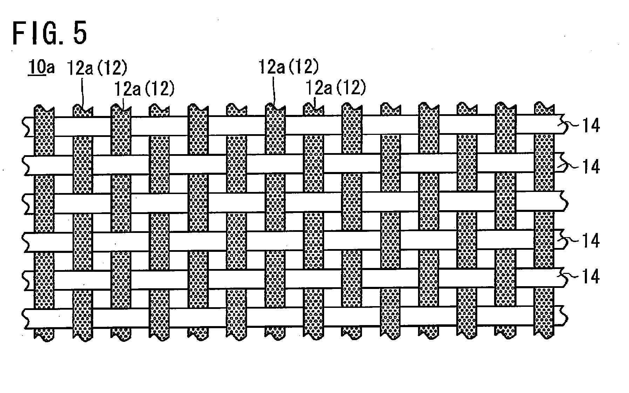

   Fig. 5 is a partly enlarged side view of the stent graft which is the first modification of the stent graft shown in Fig. 2.
[Fig. 6]

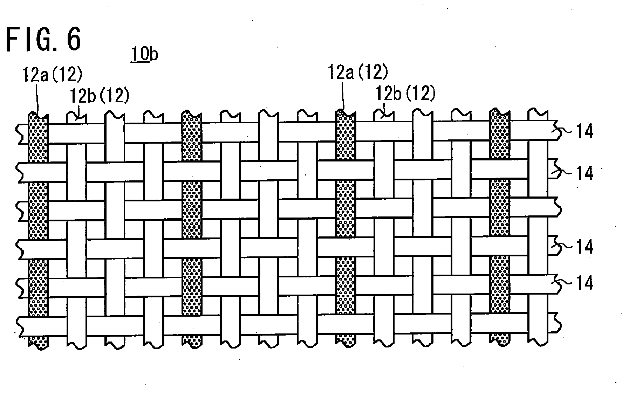

   Fig. 6 is a partly enlarged side view of the stent graft which is the second modification of the stent graft shown in Fig. 2.
[Fig. 7]

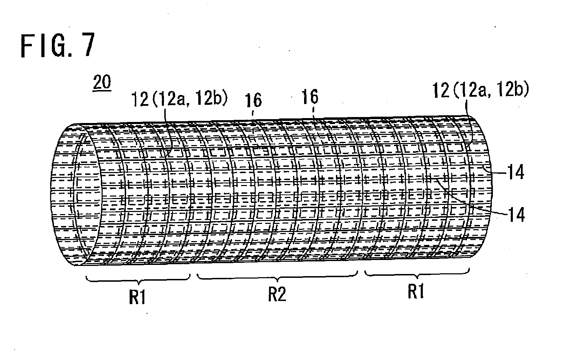

   Fig. 7 is a schematic perspective view showing the structure of a stent graft, which is the repairing material for lumens of a living body pertaining to the second embodiment of the present invention.
[Fig. 8]

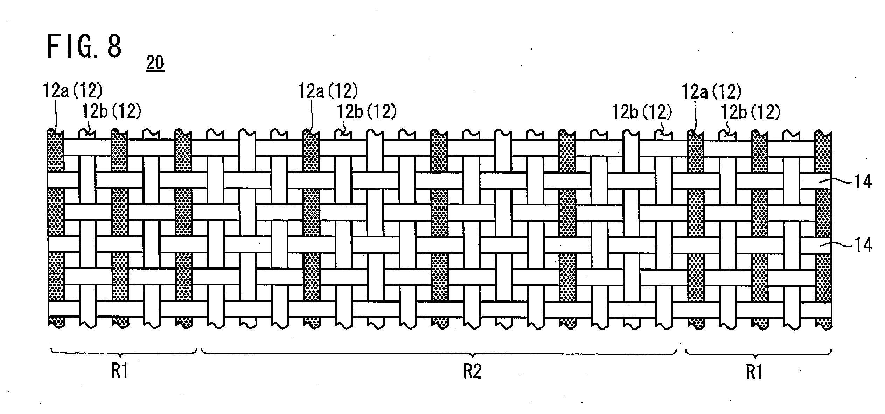

   Fig. 8 is a partly enlarged side view of the stent graft shown in Fig. 7.
[Fig. 9]

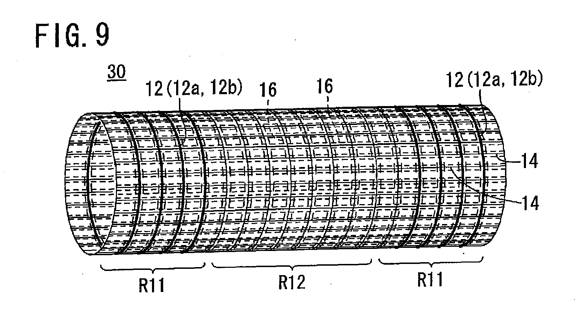

   Fig. 9 is a schematic perspective view showing the structure of a stent graft, which is the repairing material for lumens of a living body pertaining to the third embodiment of the present invention.
[Fig. 10]

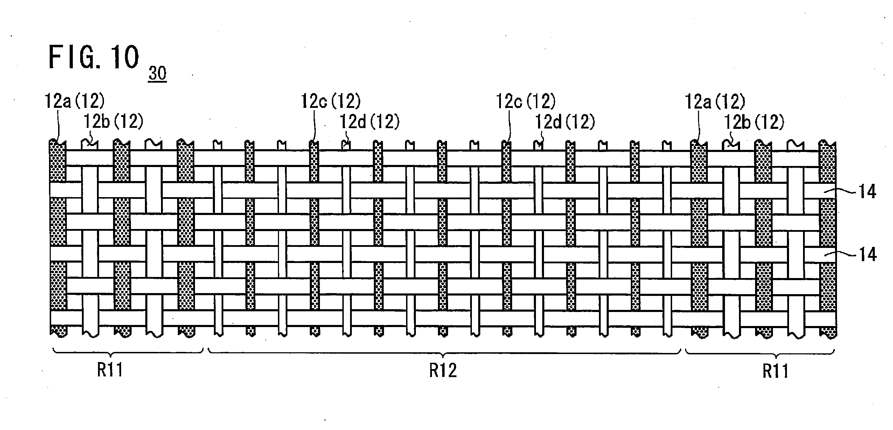

   Fig. 10 is a partly enlarged side view of the stent graft shown in Fig. 9.
[Fig. 11]

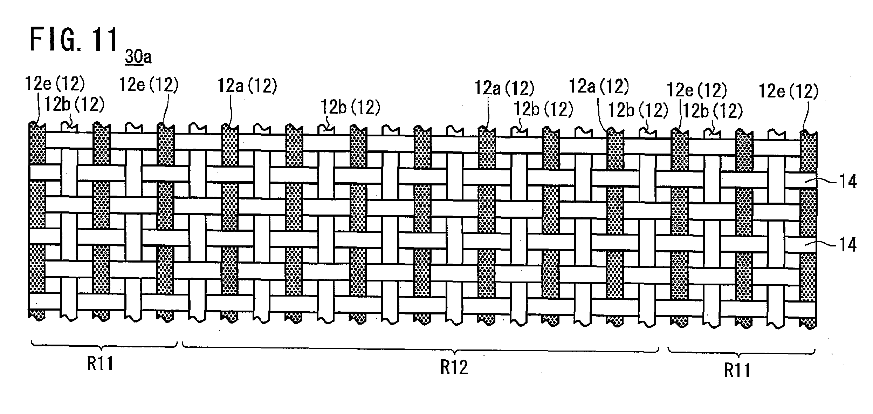

   Fig. 11 is a partly enlarged side view of the stent graft which is a modification of the stent graft shown in Fig. 10.
[Fig. 12]

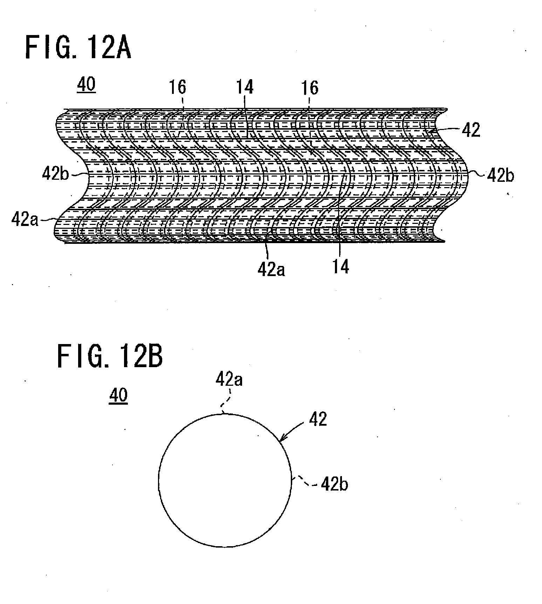

   Fig. 12A is a schematic side view showing the structure of a stent graft, which is the repairing material for lumens of a living body pertaining to the fourth embodiment of the present invention. Fig. 12B is a front view seen in the axial direction of the stent graft shown in Fig. 12A.
[Fig. 13]

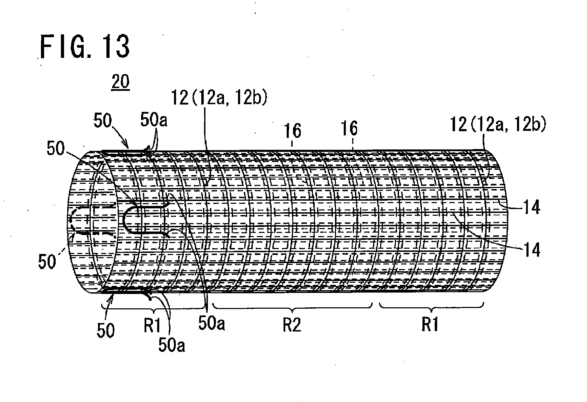

   Fig. 13 is a schematic perspective view showing the structure of the stent graft which is a modification of the stent graft shown in Fig. 7.

### Best Mode for Carrying Out the Invention

The repairing material for lumens of a living body according to the present invention will be described below with reference to the preferred embodiments and the accompanying drawings.

Fig. 1A is a perspective view showing a stent graft 10 in its expanded state, which is the repairing material for lumens of a living body pertaining to the first embodiment of the present invention. Fig. 1B is a perspective view showing the stent graft 10 shown in Fig. 1A in its folded state. The stent graft 10 pertaining to this embodiment is a blood vessel repairing material which is used for treatment of aortic aneurysm in the chest or abdomen by insertion and placement in the affected blood vessel. The repairing material according to the present invention may also be used for treatment of lumens in a living body such as trachea, esophagus, bile duct, and urethra, in addition to blood vessels.

As shown in Fig. 1A, the stent graft 10 is a tubular fabric composed of interwoven warp yarns 14 and weft yarns 12. The warp yarns 14 extend in the axial direction of the tube and the weft yarns 12 extend in the circumferential direction of the tube. The stent graft 10 is formed by interweaving the warp yarns 14 and the weft yarns 12 in the usual way for plain weaving.

The warp yarns 14 and weft yarns 12 have a swellable coating 16 on the surface thereof. The swellable coating 16 is a polymeric material capable of swelling by the body fluid (such as blood) at that part of the blood vessel which needs repair. It may exist on either of the warp yarns 14 or the weft yarns 12.

Fig. 2 is a partly enlarged side view of the stent graft 10 shown in Fig. 1A. It schematically shows a portion of the outside of the stent graft 10. It is to be noted from Fig. 2 that there exist interstices between the warp yarns 14 and the weft yarns 12, between the adjacent warp yarns 14, and between the adjacent weft yarns 12. In fact, however, the warp yarns and weft yarns are closely interwoven so that there exist substantially no interstices between them. This is apparent from the fact that the stent graft 10 constitutes a passage for the body fluid such as blood to flow therethrough.

As shown in Fig. 2, the weft yarns 12 is composed of two kinds of yarns, or yarns 12a of the first kind (indicated by shading) and yarns 12b of the second kind. The yarns 12a and 12b of the first and second kinds are arranged alternately and interwoven with the warp yarns 14, so that the stent graft 10 is formed. The yarns 12a of the first kind are more resilient that the yarns 12b of the second kind. They may be formed from shape-memory plastic, shape-memory alloy, or super-elastic metal, for instance.

The raw material for the yarns 12a of the first kind is exemplified as follows. The shape-memory plastic includes styrene-butadiene copolymer, polynorbornene, transisoprene, and polyurethane, and polymeric materials containing any one of them. The shape-memory alloy includes Ti-Ni alloy and ferroalloy. The super-elastic metal includes high-tensile stainless steel, Ti-Ni alloy containing 49 to 53 at% of Ni, Cu-Zn alloy containing 38.5 to 41.5 wt% of Zn, Cu-Zn-X alloy containing 1 to 10 wt% of X = Be, Si, Sn, Al, or Ga, and Ni-Al alloy containing 36 to 38 at% of Al. The Ti-Ni alloy may be replaced by Ti-Ni-X alloy modified with 0.01 to 10.0% of additional element such as Co, Fe, Mn, Cr, V, Al, Nb, W, and B, or by Ti-Ni-X alloy modified with 0.01 to 30.0 %at of additional element such as Cu, Pb, or Zr. The Ti-Ni alloy may have their mechanical properties properly changed by cold working and (or) heat treatment under adequate conditions. Further, with use of the Ti-Ni-X alloy, the mechanical properties can be properly changed by cold working and (or) heat treatment under adequate conditions. The super-elastic alloy should preferably be one which has a buckling strength (yield strength under load) of 5 to 200 kg/mm², preferably 8 to 150 kg/mm² (at 22°C), and a restoring force (yield strength without load) of 3 to 180 kg/mm², preferably 5 to 130 kg/mm² (at 22°C). The super-elastic metal denotes a metal which, when deformed (bent, stretched, or compressed) to such an extent that an ordinary metal undergoes plastic deformation at the temperature of use, restores its original shape almost completely, without requiring heat, after it is freed of deformation. The shape-memory plastic, shape-memory alloy, or super-elastic metal constituting the yarns 12a of the first kind should have sufficient elasticity at least at the temperature of the living body (about 37°C).

The weft yarns 12b of the second kind are formed from a plastic material, such as polyester, ePTFE (elongated polytetrafluoroethylene), and polyamide.

The warp yarns 14 are also formed from almost the same material as the one for the weft yarns 12b of the second kind, such as polyester, ePTFE (elongated polytetrafluoroethylene), and polyamide.

The weft 12 may be composed of the yarns 12a of the first kind and the yarns 12b of the second kind, which are arranged alternately one by one. In this case, the weft yarns 12a of the first kind should have a diameter of about 0.01 to 0.2 mm, preferably about 0.13 mm, and the weft yarns 12b of the second kind should have a diameter of about 0.01 to 0.3 mm, preferably about 0.05 mm. The warp yarns 14 should have a diameter of about 0.01 to 0.3 mm, preferably about 0.05 mm. Moreover, the stent graft 10 formed by weaving from the warp yarns 14 and the weft yarns 12 is a tube which is about 6 to 46 mm in outside diameter and about 40 to 200 mm in length.

According to the embodiment mentioned above, the stent graft 10 in a tubular shape is formed by interweaving the warp yarns 14 of plastic material and the weft yarns 12a of the first kind and the weft yarns 12b of the second kind, with the former being formed from any one of shape-memory plastic, shape-memory alloy, or super-elastic metal, and the latter being formed from a plastic material. In other words, the stent graft 10 has the weft yarns 12a of the first kind, which have sufficient resilience, as the weft yarns 12 interwoven in the circumferential direction. This structure obviates the necessity of attaching the skeleton (stent) to the tubular fabric unlike the conventional stent graft. Thus, the resulting stent graft is sufficiently expandable even though it is composed substantially of the tubular fabric alone.

The absence of the skeleton makes the stent graft 10 smaller in thickness and diameter than the conventional one as much as the thickness of the skeleton and the thickness of the thread to stitch the skeleton to the fabric. Therefore, the stent graft 10 can be folded up into a sufficiently small body as shown in Fig. 1B when it is inserted into a living body. The tightly folded stent graft 10 is less invasive to the patient, and it expands smoothly and completely after placement in the lumen, as shown in Fig. 1A, due to the elastic force of the weft yarns 12a of the first kind. In addition, the weft yarns 12 (or the weft yarns 12a of the first kind) which function as the skeleton exert a uniform force to the stent graft 10, which prevents the fabric from breaking and ensures the safety of the stent graft 10 in contact with the wall of the blood vessel owing to the local strong force. Moreover, because of the absence of the skeleton, the fabric for the stent graft 10 can be woven from the weft yarns 12 which are not restricted in material, dimensions, and shape, with the weaving angle unrestricted. This facilitates the production of the stent graft 10 suitable for its applications and specifications.

The stent graft 10 pertaining to this embodiment is woven from the warp yarns 14 and the weft yarns 12 which have the swellable coating 16 on the surface thereof. The swellable coating 16 results in the reduction of the number of the warp yarns 14 and the weft yarns 12 as compared with the case where the swellable coating 16 is not provided. This helps reduce more the diameter of the stent graft at the time of folding. The warp yarns 14 and weft yarns 12 in reduced number would result in some interstices between yarns before insertion into a living body; however, such interstices are clogged because the swellable coating 16 swells with the body fluid after the stent graft 10 has expanded in the lumen. Thus, there is no possibility of the stent graft impairing in function. In other words, the warp yarns 14 and weft yarns 12 having the swellable coating 16 reduce the water permeability of the fabric of the stent graft 10, which helps reduce the weaving density and permits the stent graft 10 to be folded up into a much smaller body.

It is also possible to produce almost the same effect as mentioned above even though the warp yarns 14 and weft yarns 12 are made of a plastic material capable of swelling in place of those having the swellable coating 16. It is also possible to apply the swellable coating 16 to only either of the warp yarns 14 or the weft yarns 12. In this case, too, the resulting stent graft 10 can be folded up into a small body to a certain extent.

Incidentally, the stent graft 10 shown in Fig. 2 is formed from a fabric of plain weave composed of the warp yarns 14 and the weft yarns 12 which are interwoven such that each weft yarn passes successively over and under each warp yarn. However, the stent graft may be formed from a fabric of any other type, as a matter of course. An example is a twill weave, in which the warp yarn 14 successively passes over and under two weft yarns 12 (one weft yarn 12a of the first kind and one weft yarn 12b of the second kind), as shown in Fig. 3. Another example is a satin weave, in which the warp yarn 14 successively passes over and under four weft yarns 12 (two weft yarns 12a of the first kind and two weft yarns 12b of the second kind), as shown in Fig. 4. Any other known weaving system may be employed.

The stent graft 10 illustrated above is one in which the weft yarns 12 are composed of the yarns 12a of the first kind and the yarns 12b of the second kind. However, it may be replaced by the stent graft 10a in which the weft yarns 12 are composed only of the elastic fibers 12a of the first kind, as shown in Fig. 5.

In the stent graft 10a, the weft yarns 12 may be composed entirely of the yarns 12a of the first kind which are metallic yarns. As compared with the stent graft 10 in which the weft yarns are the yarns 12b of the second kind made of plastic, the stent graft 10a is more likely to cause the adjacent yarns 12a of the first kind to slip from each other. However, the stent graft 10a is easier to produce because it does not employ the yarns 12b of the second kind. This is desirable for some applications and specifications. On the other hand, the stent graft 10a may be one in which the weft yarns are composed of the yarns 12a of the first kind made of shape-memory plastic. This structure prevents the adjacent yarns 12a of the first kind from slipping from each other. The same effect as above may be produced when the yarns 12a of the first kind which are made of shape-memory plastic and super-elastic metal (or shape-memory alloy) are arranged alternately.

In the case of the stent graft 10a in which the weft yarns 12 are composed entirely of the yarns 12a of the first kind, the weft yarns 12 should be ones having a diameter of about 0.01 to 0.15 mm, preferably about 0.1 mm. The weft yarns 12 of this size prevents the resulting stent graft 10a from having excessively large elasticity and resistance to compression. Thus, the stent graft 10a has almost the same properties as the stent graft 10 which employs the yarns 12b of the second kind.

The stent graft 10 shown in Fig. 1A and others is constructed such that the weft yarns 12 are composed of the yarns 12a of the first kind and the yarns 12b of the second kind in a ratio of 1:1 which are arranged alternately. The ratio of the yarns 12a and 12b may be varied as desired as the matter of course.

For example, in the case of the stent graft 10b shown in Fig. 6, the weft yarns 12 are composed of the yarns 12a of the first kind and the yarns 12b of the second kind in a ratio of 1:3 which are arranged alternately. In other words, one out of the four weft yarns 12 is the yarn 12a of the first kind. In this case, the weft yarns 12 (or the yarn 12a of the first kind and the yarn 12b of the second kind) should have a diameter of about 0.02 to 0.23 mm, preferably about 0.16 mm. The yarns of this diameter ensure sufficient resilience even though their ratio is lower than that (1:1) mentioned above.

Needless to say, the weft yarns 12 may be composed of the yarns 12a of the first kind and the yarns 12b of the second kind in any other ratio than mentioned above. For example, the ratio of the yarns 12a of the first kind and the yarns 12b of the second kind may be 1:7, in which case one out of the eight weft yarns 12 is the yarn 12a of the first kind. In this case, the weft yarns 12 (or the yarn 12a of the first kind and the yarn 12b of the second kind) should have a diameter of about 0.02 to 0.29 mm, preferably about 0.2 mm. Likewise, the ratio of the yarns 12a of the first kind and the yarns 12b of the second kind may be 1:15, in which case one out of the sixteen weft yarns 12 is the yarn 12a of the first kind. In this case, the weft yarns 12 (or the yarn 12a of the first kind and the yarn 12b of the second kind) should have a diameter of about 0.02 to 0.3 mm, preferably about 0.22 mm. Moreover, the ratio of the yarns 12a of the first kind and the yarns 12b of the second kind may be 1:31, in which case one out of the 32 weft yarns 12 is the yarn 12a of the first kind. In this case, the weft yarns 12 (or the yarn 12a of the first kind and the yarn 12b of the second kind) should have a diameter of about 0.02 to 0.3 mm, preferably about 0.3 mm.

As mentioned above, it is possible to properly change the ratio of the yarns 12a of the first kind and the yarns 12b of the second kind which constitute the weft yarns 12 over a wide range from 1:0 (or 1:1) to 1:31. The number of the yarns 12a of the first kind may be two or more, as the matter of course; the adequate ratio depends on the applications and specifications of the stent graft 10 (10a and 10b). The stent graft will have adequate expandability and compressibility according as the yarns 12a of the first kind (which are resilient) and the yarns 12b of the second kind are arranged alternately in a specific ratio.

The foregoing illustration demonstrates that the stent graft 10 exhibits sufficient resilience when the yarns 12a of the first kind increase in diameter as their ratio decreases, even in the case where the elastic yarns 12a of the first kind exist in a small ratio. For the folded stent graft to expand, or restore its original shape, by the elastic force of the yarns 12a of the first kind, the yarns 12a of the first kind made of the above-mentioned material should preferably have a diameter up to about 0.3 mm. Of course, it is not necessary to change the diameter if the yarns 12a of the first kind are made of an adequate material so that they exhibit sufficient resilience.

The above-mentioned ratio of the yarns 12a of the first kind and the yarns 12b of the second kind may be constant over the entire length of the stent grafts 10 and 10b, and the stent grafts 30, 30a, and 40 (mentioned later). This prevents more certainly the yarns 12a of the first kind (made of metal) from lying side by side and slipping from each other. Thus the stent graft 10 exhibits expandability and compressibility stably and uniformly over the entire length thereof.

Fig. 7 is a schematic perspective view showing the structure of the stent graft 20, which is the repairing material for lumens of a living body pertaining to the second embodiment of the present invention. Fig. 8 is a partly enlarged side view of the stent graft 20 shown in Fig. 7. Incidentally, the stent graft 20 pertaining to the second embodiment has some constituents in common with the stent graft 10 (10a and 10b) pertaining to the first embodiment, so that they produce identical functions and effects, and such constituents are referenced by the same numerals without repetition of their detailed description. The same shall apply hereinafter.

The stent graft 20 shown in Figs. 7 and 8 is constructed such that the ratio (density) of the yarns 12a of the first kind to the yarns 12b of the second kind both constituting the weft yarns 12 is higher near the ends R1 and R1 thereof than at the intermediate part R2 thereof. Fig. 8 demonstrates an example in which the ratio of the yarns 12a of the first kind to the yarns 12b of the second kind is 1:1 near the ends R1 and R1 and 1:3 at the intermediate part R2.

The stent graft 20, in which the ratio is higher near the ends R1 thereof, exhibits stronger resilience near the ends R1 thereof and weaker resilience at the intermediate part R2 thereof. Therefore, the stent graft 20 strongly expands at the ends R1 and R1 thereof when it is placed at the desired position in the lumen and smoothly expands as a whole in the lumen. This ensures the placement of the stent graft 20 in the lumen. Moreover, the stent graft 20, which has a comparatively weak expanding force at the intermediate part R2 thereof, can be easily folded up owing to the adequately reduced compressive resistance (see Fig. 1B). It can also be easily released from the sheath of a catheter (not shown) owing to its adequately reduced rebound resilience.

Needless to say, the stent graft 20 does not necessarily have the above-mentioned ratio for the yarns 12a of the first kind and the yarns 12b of the second kind. It is only necessary that the ratio be higher near the ends R1 and R1 than at the intermediate part R2.

The stent graft 20 should have the end parts R1 and R1 extending about 5 to 25 mm, preferably about 10 mm, from the end thereof, in which the ratio of the yarns 12a of the first kind to the yarns 12b of the second kind is high (say, 1:1), with the number of the yarns 12a of the first kind being at least two. The point is that the extent of the end part should be long enough for both smooth expansion and low compressive resistance in compliance with the applications and specifications of the stent graft 20.

In addition, the stent graft 20 may be constructed such that at least either of its ends has the yarn 12a of the first kind, as shown in Fig. 8. The yarn 12a of the first kind at the end exerts a sufficient expanding force, thereby ensuring the placement of the stent graft 20 in the lumen, such as blood vessel. Moreover, the stent graft 20 should be placed in the lumen in such a way that the end thereof in which the yarn 12a of the first kind is arranged orients upward (headward, upstream) in the lumen. This ensures the placement of the stent graft 20 in the lumen (blood vessel) because the upper end exerts a sufficient expanding force. The structure having the yarn 12a of the first kind in either end may be applied also to the stent grafts 10, 10a, 10b, 30, 30a, and 40 pertaining to other embodiments.

Fig. 9 is a schematic perspective view showing the structure of the stent graft 30, which is the repairing material for lumens of a living body pertaining to the third embodiment of the present invention. Fig. 10 is a partly enlarged side view of the stent graft 30 shown in Fig. 9.

The stent graft 30 shown in Figs. 9 and 10 is constructed such that the weft yarns 12 vary in diameter in going from the ends R11 and R11 near the ends in the axial direction to the intermediate part R12. Incidentally, the end R11 and the intermediate part R12 range over the same length as the end R1 and the intermediate part R2 of the stent graft 20 mentioned above.

The stent graft 30 shown in Fig. 10 employs the yarns 12a of the first kind and the yarns 12b of the second kind in a ratio of 1:1. In this stent graft 30, the intermediate part R12 is composed the yarns 12c of the first kind and the yarns 12d of the second kind, and the ends R11 and R11 are composed of the yarns 12a of the first kind and the yarns 12b of the second kind. It is to be noted that 12c and 12d are thinner than 12a and 12b. As in the case of the stent graft 20 pertaining to the second embodiment, the stent graft 30 exerts a stronger resilience near the ends thereof and a weaker resilience at the intermediate part thereof.

The stent graft 30 is characterized in that the weft yarns constituting the intermediate part R12 are thinner than those constituting the end R11. This structure causes the tube to exert a stronger resilience near the ends and a weaker resilience at the intermediate part. The same effect will be produced by replacing it with the stent graft 30a shown in Fig. 11.

In the stent graft 30a, the end R11 and the intermediate part R12 are constructed of the weft yarns which are the same in diameter but different in resilience. That is, the weft yarns 12e of the first kind for the end R11 are more resilient than the weft yarns 12a of the first kind for the intermediate part R12. Consequently, the stent graft 30a also produces the same effect as the stent grafts 20 and 30. In other words, it exhibits stronger resilience near the end R11 than at the intermediate part R12.

The stent grafts 20, 30, and 30a mentioned above are so constructed as to exhibit stronger resilience near the ends of the tube. However, in the case of stent grafts with a long tube length, this structure may be modified such that it exerts stronger resilience near the ends and middle part and weaker resilience at the intermediate part thereof.

Fig. 12A is a schematic side view showing the structure of the stent graft 40, which is the repairing material for lumens of a living body pertaining to the fourth embodiment of the present invention. Fig. 12B is a front view seen in the axial direction of the stent graft 40 shown in Fig. 12A.

As shown in Figs. 12A and 12B, the stent graft 40 is formed by interweaving the warp yarns 14 and weft yarns 42 in place of the weft yarns 12 which curve in a wavy shape in the lengthwise direction even after it has expanded.

As in the case of the stent graft 10 mentioned above and shown in Fig. 1B, the stent graft 40 forms at least a pair of projected parts and a pair of retracted parts at the ends thereof when it is folded up. With this taken into consideration, the weft yarns 42 should take on a wavy shape with at least two each of convexes and concaves projecting and retracting in the axial direction as viewed from the side. This structure facilitates the folding work. It also offers the advantage that the stent graft 40 undergoes a less amount of deformation due to compression than the stent graft 10 shown in Fig. 1 when it is folded up for insertion into the sheath of a catheter (not shown) from the expanded state shown in Fig. 12A to the compressed state as shown in Fig. 1B. As the result, the weft yarns 42 effectively decrease in rebound resilience in the sheath, and this permits the stent graft 10 to be released more easily from the sheath.

The stent graft 40, which takes on a wavy shape in its expanded state, forms the skeleton in the axial direction which is absent in the stent graft 10 formed by plain weaving as shown in Fig. 1A (the skeleton consists of the ridge 42a and the trough 42B both running in the axial direction). This structure produces a larger resistance to compression in the axial direction than the structure of the stent graft 10.

In addition, the skeleton mentioned above, which has a certain length in the axial direction, effectively prevents the stent graft 40 from being placed aslant in the blood vessel.

Needless to say, the weft yarns 42 taking on the wavy shape can be applied to the above-mentioned stent grafts 10, 10a, 10b, 20, 30, and 30a.

The foregoing embodiments are not intended to restrict the scope of the present invention. The present invention may be variously modified within the scope thereof.

For example, the stent graft 20 pertaining to the second embodiment may be modified by providing it with a plurality of hooks (thorns) 50 around the periphery at one end. The result of such modification is the stent graft 20a (which has four hooks) shown in Fig. 13. The hook 50 takes on a U-shape, with its two ends 50a and 50a orienting to the other end of the stent graft 20a and bending and projecting outward.

The hooks 50 are attached to that end of the stent graft 20a which orients upward (headward, upstream) in the lumen during its use. Thus, their fore-ends bite into the vessel wall, thereby preventing the stent graft 20a from being displaced. Moreover, the hooks 50 are attached to the region R1 of the stent graft 20a where the expanding force is greatest, so that they effectively prevent the stent graft 20a from being displaced.

Needless to say, the hooks 50 may be applied to the above-mentioned stent grafts 10, 10a, 10b, 30, 30a, and 40.

## Claims

1. A repairing material (10, 10a, 10b, 20, 30, 30a, 40) for lumens of a living body comprising:
warp yarns (14) of plastic material, and
weft yarns (12, 42) including filaments of at least one of shape-memory plastic, shape-memory alloy, and super-elastic metal,
said warp yarns and weft yarns being interwoven into a tubular shape such that said weft yarns (12, 42) extend in the circumferential direction and said warp yarns (14) extend in the axial direction,
with either or both of said warp yarns (14) and weft yarns (12, 42) being formed from yarns capable of swelling by body liquid or from yarns with coating (16) capable of swelling by body fluid.

2. The repairing material (40) for lumens of a living body as defined in Claim 1, wherein
said weft yarns (42) are interwoven to form a tube whose cross section has an irregular wavy shape in the axial direction composed of at least two ridges (42a) and at least two troughs (42b) extending in the circumferential direction.

3. The repairing material (10, 10b, 20, 30, 30a) for lumens of a living body as defined in Claim 1, wherein
the weft yarns (12) are composed of two kinds of yarns, with the first one (12a, 12c, 12e) being formed from at least one of shape-memory plastic, shape-memory alloy, and super-elastic metal, and the second one (12b, 12d) being formed from a plastic material,
said weft yarns (12a, 12c, 12e) of the first kind and said weft yarns (12b, 12d) of the second kind being interwoven alternately in a prescribed ratio with the warp yarns (14).

4. The repairing material (10, 10b, 20, 30, 30a) for lumens of a living body as defined in Claim 3, wherein
the mixing ratio of the weft yarns (12a, 12c, 12e) of the first kind and the weft yarns (12b, 12d) of the second kind is such that they are arranged alternately in a ratio of one (for 12a, 12c, 12e) to one (for 12b, 12d) or one (or more) (for 12a) to two (or more) (for 12b).

5. The repairing material (10, 10b, 30, 30a) for lumens of a living body as defined in Claim 3, wherein
the ratio of weft yarns (12a, 12c, 12e) of the first kind to the weft yarns (12b, 12d) of the second kind is maintained over the entire length of the repairing material (10, 10b, 30, 30a) for lumens of a living body.

6. The repairing material (20) for lumens of a living body as defined in Claim 3, wherein
the ratio of the weft yarns (12a) of the first kind to the weft yarns (12b) of the second kind is higher near the ends (R1) than at the intermediate part (R2) in the axial direction of the repairing material (20) for lumens of a living body.

7. The repairing material (20, 30, 30a) for lumens of a living body as defined in Claim 3, wherein
at least either of the ends of the repairing material (20, 30, 30a) for lumens of a living body is formed from the weft yarns (12a, 12e) of the first kind.

8. The repairing material (30, 30a) for lumens of a living body as defined in Claim 1, wherein
the weft yarns (12) arranged near the ends (R11) thereof are more resilient than the weft yarns (12) arranged at the intermediate part (R12) thereof, said ends and intermediate part being in the axial direction of the repairing material (30, 30a) for lumens of a living body.

9. The repairing material (30, 30a) for lumens of a living body as defined in Claim 8, wherein
the weft yarns (12) arranged near the ends (R11) are composed of yarns (12a) thicker than the weft yarns (12) arranged at the intermediate part (R12), or yarns (12e) which are more resilient than the weft yarns (12) arranged at the intermediate part (R12), said ends and intermediate part being in the axial direction of the repairing material (30, 30a) for lumens of a living body.

10. The repairing material (10, 10a, 10b, 20, 30, 30a, 40) for lumens of a living body as defined in Claim 1, wherein
said repairing material (10, 10a, 10b, 20, 30, 30a, 40) for lumens of a living body has more than one hook (50) on the circumference of at least one end in the axial direction of said repairing material (10, 10a, 10b, 20, 30, 30a, 40) for lumens of a living body.
